(19) Europäisches Patentamt
European Patent Office
Office européen des brevets

(11) Numéro de publication : **0 522 914 A1**

# (12) DEMANDE DE BREVET EUROPEEN

(21) Numéro de dépôt : **92401770.0**

(22) Date de dépôt : **24.06.92**

(51) Int. Cl.$^5$ : **C07D 239/42,** C07D 401/04, C07D 401/12, A61K 31/505

(30) Priorité : **27.06.91 FR 9107938**
**18.05.92 FR 9206004**

(43) Date de publication de la demande :
**13.01.93 Bulletin 93/02**

(84) Etats contractants désignés :
**AT BE CH DE DK ES FR GB GR IT LI LU MC NL PT SE**

(71) Demandeur : **SYNTHELABO**
**22, Avenue Galilée**
**F-92350 Le Plessis Robinson (FR)**

(72) Inventeur : **George, Pascal**
**19, rue des Quatre Vents**
**F-78730 St Arnoult en Yvelines (FR)**
Inventeur : **Maloizel, Christian**
**21, rue du Plateau**
**F-92190 Meudon (FR)**
Inventeur : **Marabout, Benoit**
**6, rue Georges Ritz**
**F-91300 Massy (FR)**
Inventeur : **Merly, Jean-Pierre**
**11, avenue Jules Guesde**
**F-92330 Sceaux (FR)**

(74) Mandataire : **Ludwig, Jacques et al**
**SYNTHELABO, Service Brevets, 22, avenue Galilée, B.P. 72**
**F-92352 Le Plessis Robinson Cédex (FR)**

## (54) Dérivés de 2-pipéridinylpyrimidine-4-carboxamide, leur préparation et leur application en thérapeutique.

(57) Composé répondant à la formule générale (I)

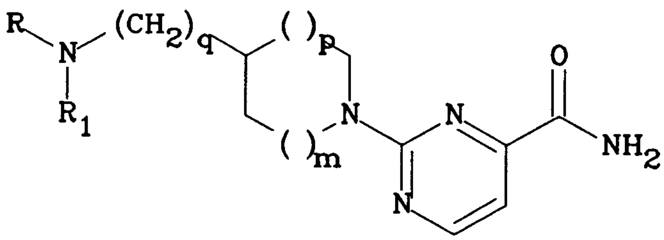

(I)

dans laquelle R représente un atome d'hydrogène ou un groupe méthyle ou un groupe phénoxyalkyle de formule générale

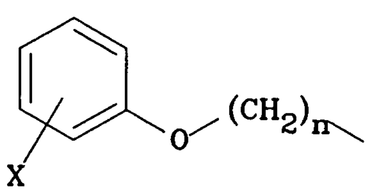

(dans laquelle X représente un ou plusieurs substituants choisis parmi l'hydrogène, le fluor et le chlore et les groupes méthyle, 1-méthyléthyle et méthoxy, et n=2 ou 3), m=1, auquel cas p=1, ou bien m=0, auquel cas p=2, q=0 ou 1, et $R_1$ représente un atome d'hydrogène ou un groupe méthyle.
Application en thérapeutique.

EP 0 522 914 A1

La présente invention a pour objet des dérivés de 2-pipéridinylpyrimidine-4-carboxamide, leur préparation et leur application en thérapeutique.

Les composés de l'invention répondent à la formule générale (I)

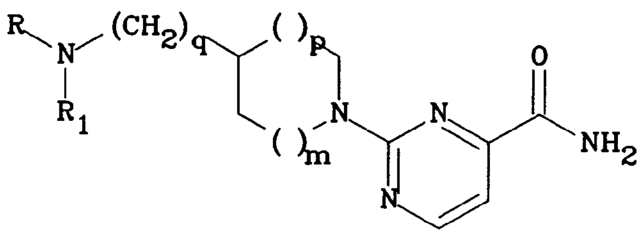

(I)

dans laquelle

R représente un atome d'hydrogène ou un groupe méthyle ou un groupe phénoxyalkyle de formule générale

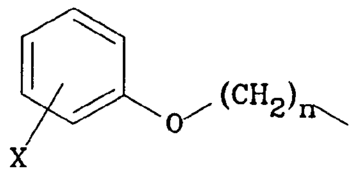

dans laquelle

X représente un ou plusieurs substituants choisis parmi les atomes d'hydrogène, de fluor et de chlore et les groupes méthyle, 1-méthyléthyle et méthoxy, et

n représente le nombre 2 ou 3,

m représente le nombre 1, auquel cas p représente le nombre 1, ou bien

m représente le nombre 0, auquel cas p représente le nombre 2,

q représente le nombre 0 ou 1, et

$R_1$ représente un atome d'hydrogène ou un groupe méthyle.

Les composés de l'invention peuvent se présenter à l'état de bases libres ou de sels d'addition à des acides, ainsi que sous forme d'énantiomères purs ou de mélanges d'énantiomères, par exemple racémiques.

Conformément à l'invention, on peut préparer les composés de formule générale (I) selon l'un des procédés illustrés par les schémas 1 et 2 qui suivent.

Le schéma 1 concerne seulement les composés de formule générale (I) dans laquelle m = p = 1 et q = 0, ce qui correspond à la formule générale (I').

Schéma 1

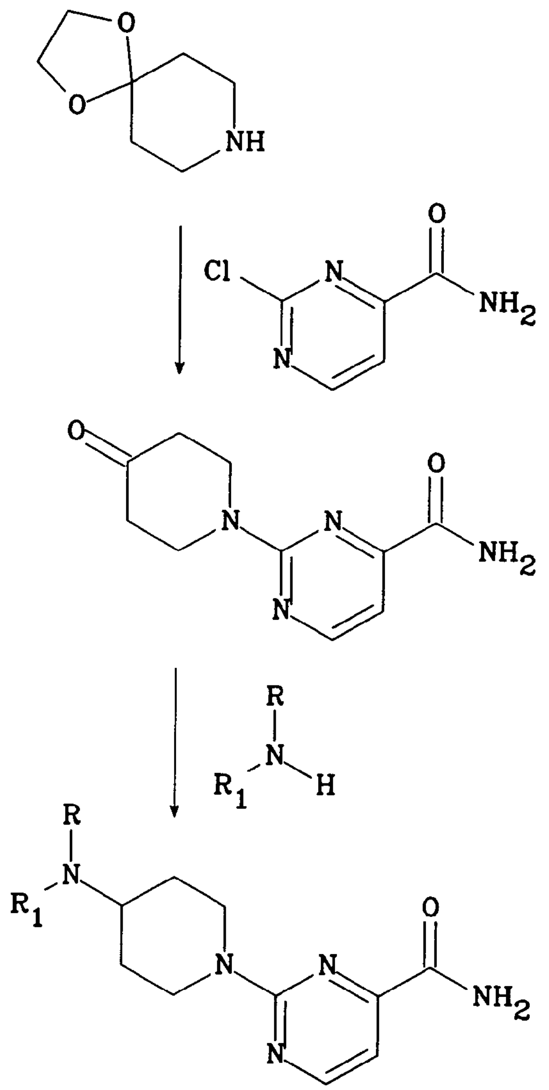

(II')

(III)

(IV')

(V')

(I')

Selon ce schéma, on fait réagir la pipéridine substituée de formule (II′) avec le 2-chloropyrimidine-4-carboxamide de formule (III) dans un solvant aprotique, par exemple l'acétonitrile, en présence d'une base minérale, par exemple le carbonate de potassium. On transforme l'acétal obtenu en cétone de formule (IV′) par hydrolyse, par exemple avec de l'acide chlorhydrique.

On obtient ensuite un dérivé de formule générale (I′)

- soit en une étape, par amination réductive de la cétone de formule (IV′) avec un cyanoborohydrure alcalin, par exemple le cyanoborohydrure de lithium, dans un alcool, par exemple le méthanol, en présence d'acétate d'ammonium, en maintenant le pH de la solution entre 5 et 6 ;
- soit en deux étapes, par réaction de la cétone de formule (IV′) avec une amine de formule générale (V′), dans laquelle R et $R_1$ sont tels que définis ci-dessus, dans un solvant tel que le dichlorométhane, en présence d'un agent déshydratant tel de le sulfate de magnésium, puis réduction de l'imine intermédiaire par un hydrure alcalin, par exemple le borohydrure de sodium, dans un solvant tel que l'éthanol.

En variante, on peut obtenir un composé de formule générale (I), dans laquelle R représente un groupe phénoxyalkyle tel que défini ci-dessus, à partir d'un composé de formule générale (I) dans laquelle R représente l'hydrogène, comme décrit ci-dessous à propos de l'étape (V) → (I″) du schéma 2.

La pipéridine substituée de formule (II′), ou 1,4-dioxa-8-azaspiro[4.5]décane, est disponible dans le commerce.

Le 2-chloropyrimidine-4-carboxamide de formule (III) peut être préparé à partir de 2-chloropyrimidine-4-carbonitrile par traitement à l'acide chlorhydrique gazeux dans l'acide formique, ledit nitrile étant lui-même préparé selon la méthode décrite dans *J. Het. Chem.*, 1964, **1**, 130-133.

Selon le schéma 2 ci-dessous, on fait d'abord réagir une diamine de formule générale (II), dans laquelle $R_1$, m, p et q sont tels que définis ci-dessus et R' représente un groupe protecteur de l'amine, par exemple un groupe benzyloxycarbonyle ou un groupe t.-butoxycarbonyle, avec le 2-chloropyrimidine -4-carboxamide de formule (III), dans un solvant aprotique, par exemple le *N*,*N*-diméthylformamide, en présence d'une base, par exemple minérale, telle que le carbonate de

Schéma 2

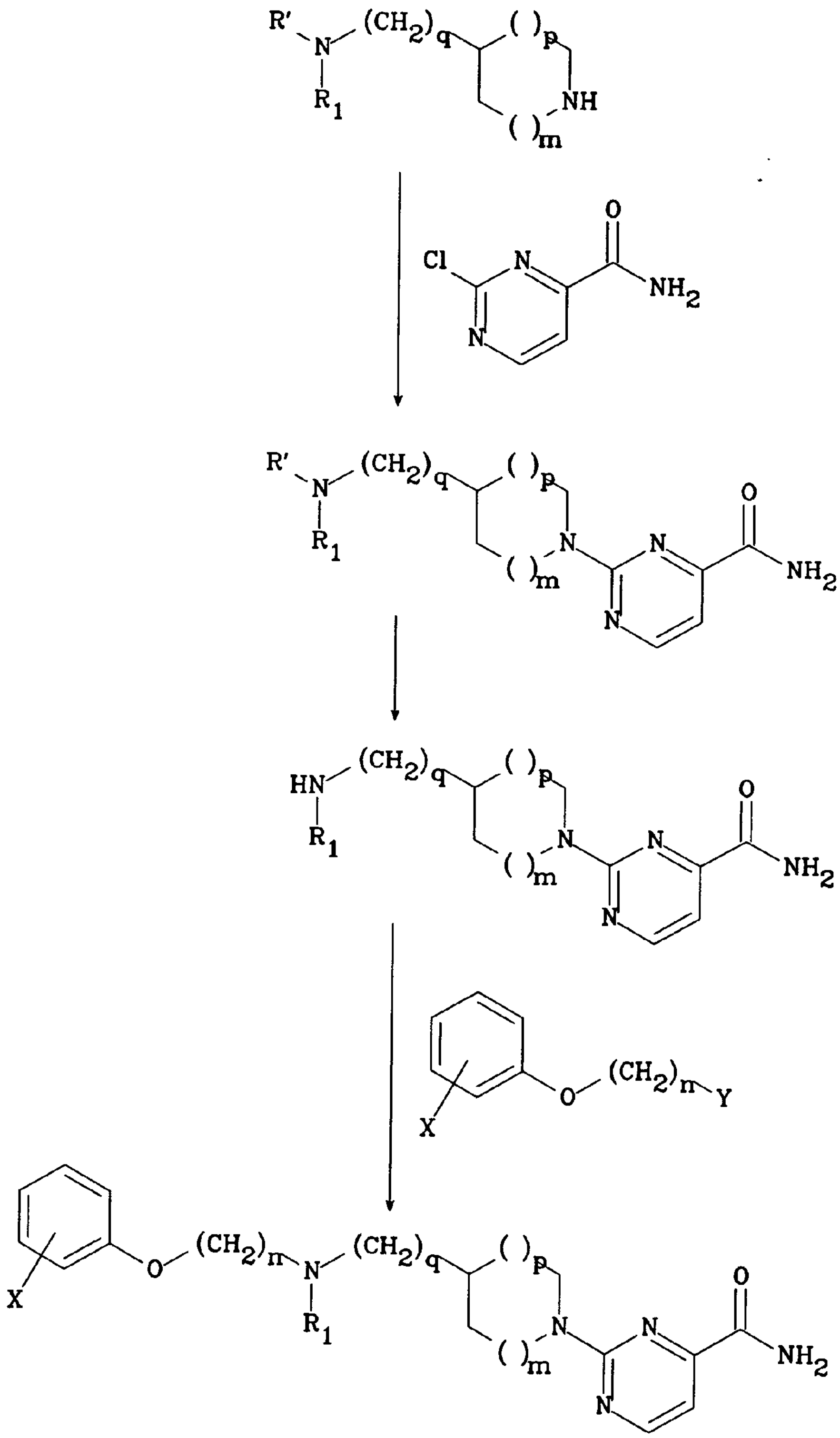

(II)

(III)

(IV)

(V)

(VI)

(I")

potassium, à une température de 20 à 40°C.

On obtient une aminopyrimidine de formule générale (IV), que l'on déprotège ensuite, selon la nature du

groupement protecteur R′, par une méthode analogue à celles décrites dans la littérature, par exemple par hydrogénation en présence de charbon palladié (dans le cas d'un groupe benzyloxycarbonyle) ou par réaction avec l'acide trifluoroacétique dans le dichlorométhane (dans le cas d'un groupe t.-butoxycarbonyle).

On obtient une aminopyrimidine de formule générale (V), qui correspond à la formule générale (I) lorsque R représente l'hydrogène.

Si on le désire, on la fait finalement réagir avec un halogénure de phénoxyalkyle de formule générale (VI), dans laquelle X et n sont tels que définis ci-dessus et Y représente un atome de chlore ou de brome, dans un solvant aprotique, par exemple le *N,N*-diméthylformamide, en présence d'une base, par exemple minérale, telle que le carbonate de potassium, à une température de 60 à 80°C.

Les diamines protégées de formule générale (II), dans laquelle q représente le nombre 1, peuvent être préparées par une méthode analogue à celles décrites pour la synthèse du pipéridine-4-carbamate de t.-butyle (q = 0) dans les demandes de brevets DE-2831431, EP-410278 et EP-417698.

Le 2-chloropyrimidine-4-carboxamide de formule (III) est décrit ci-dessus à propos du schéma 1.

Les halogénures de phénoxylalkyle de formule générale (VI) peuvent être préparés par des méthodes analogues à celles décrites dans *J. Pharm. Sci.* 1984, **73/9**, 1241-4, ou dans Synthesis 1990, 1069-71.

Les exemples suivants illustrent la préparation de quelques composés selon l'invention. Les microanalyses élémentaires et les spectres IR et RMN confirment les structures des produits obtenus. Les numéros de composés, donnés entre parenthèses dans les titres des exemples, correspondent à ceux du tableau donné plus loin.

Exemple 1 (Composé N°3)

2-[4-[[2-(2-méthoxyphénoxy)éthyl]amino]pipéridin-1-yl]pyrimidine-4-carboxamide, chlorhydrate.

1.1. 2-(1,4-dioxa-8-azaspiro[4.5]décan-8-yl)pyrimidine-4-carboxamide.

On introduit 5 g (0,0318 mole) de 2-chloropyrimidine-4-carboxamide, 4,55 g (0,0318 mole) de 1,4-dioxa-8-azaspiro[4.5]décane et 6,6 g (0,0477 mole) de carbonate de potassium dans 100 ml de butan-2-one. On chauffe et agite le mélange à la température du reflux pendant 6 heures. On filtre et concentre le filtrat sous pression réduite. On obtient, 8,2 g de produit que l'on fait recristalliser dans du propan-2-ol. On obtient 4,1 g de produit.
Point de fusion : 173-175°C.

1.2. 2-(4-oxopipéridin-1-yl)pyrimidine-4-carboxamide.

On chauffe à la température du reflux pendant 30 minutes, un mélange de 4,1 g (0,0155 mole) de 2-(1,4-dioxa-8-azaspiro[4.5]décan-8-yl)pyrimidine-4-carboxamide, 41 ml d'acide acétique et 4,1 ml d'acide chlorhydrique concentré. On concentre le mélange sous pression réduite puis reprend le résidu dans un mélange de dichlorométhane et d'eau, on alcalinise avec une solution d'ammoniaque, on extrait au dichlorométhane, on sèche la phase organique sur sulfate de magnésium, on la filtre, et on évapore le solvant sous pression réduite. On obtient 3 g de composé.
Point de fusion : 208-212°C.

1.3. 2-[4-[[2-(2-méthoxyphénoxy)éthyl]imino]pipéridin-1-yl]pyrimidine-4-carboxamide.

Dans un ballon de 500 ml on introduit 2,6 g (0,0118 mole) de 2-(4-oxopipéridin-1-yl)pyrimidine-4-carboxamide, 1,97 g (0,0188 mole) de 2-(2-méthoxyphénoxy)éthylamine, 4,26 g (0,0354 mole) de sulfate de magnésium et 152 ml de dichlorométhane et on agite le mélange à la température ambiante pendant 24 heures.
On filtre, rince le précipité avec du dichlorométhane et concentre le filtrat sous pression réduite. On obtient 4,35 g d'huile que l'on utilise telle quelle dans l'étape suivante.

1.4. 2-[4-[[2-(2-méthoxyphénoxy)éthy]amino]pipéridin-1-yl]pyrimidine-4-carboxamide, chlorhydrate.

On introduit 4,35 g (0,0118 mole) de 2-[4-[[2-(2-méthoxyphénoxy)éthyl]imino]pipéridin-1-yl]pyrimidine-4-carboxamide dans 150 ml d'éthanol. On refroidit par un bain de glace, on ajoute 1,35 g (0,0354 mole) de borohydrure de sodium et on agite le mélange pendant 18 heures.
On le concentre puis on le reprend avec un mélange d'acétate d'éthyle et d'acide chlorhydrique dilué, on lave la phase aqueuse avec de l'acétate d'éthyle, puis on alcalinise avec de l'ammoniaque et on extrait avec de l'acétate d'éthyle. On lave la phase organique à l'eau, on la sèche sur sulfate de magnésium, on la filtre et on

la concentre sous pression réduite.
On met en solution la base obtenue dans 50 ml de propan-2-ol et on ajoute 50 ml d'une solution d'acide chlorhydrique 0,1N dans du propan-2-ol. On concentre le volume de moitié et on obtient 1,2 g de composé qui cristallise.
Point de fusion : 231-233°C.

Exemple 2 (Composé N°1)

2-(4-aminopipéridin-1-yl)pyrimidine-4-carboxamide, chlorhydrate.

On met en suspension 1,1 g (0,005 mole) de 2-(4-oxopipéridin1-yl)pyrimidine-4-carboxamide et 4,63 g (0,060 mole) d'acétate d'ammonium dans 15 ml de méthanol. On ajoute 0,24 g (0,005 mole) de cyanoborohydrure de lithium, on agite le mélange à la température ambiante pendant 56 heures en maintenant le pH entre 5 et 6 par addition d'acide chlorhydrique 1N. On évapore le méthanol, on alcalinise la phase aqueuse à pH=14 avec de la soude concentrée, puis on sature avec du chlorure de sodium. On extrait au dichlorométhane, on sèche la phase organique sur sulfate de magnésium, on filtre puis évapore sous pression réduite pour obtenir 0,55 g de produit.
Point de fusion : 104-105°C.
On prépare le chlorhydrate par addition de 25 ml d'acide chlorhydrique 0,1N dans du propan-2-ol. On obtient 0,65 g de produit.
Point de fusion : 304-307°C.

Exemple 3 (Composé N°7)

2-[4-[[2-(5-fluoro-2-méthoxyphénoxy)propyl]amino]pipéridin-1-yl]pyrimidine-4-carboxamide, chlorhydrate.

Dans un ballon tricol de 100 ml, on introduit sous argon 1,7 g (0,00768 mole) de 2-(4-aminopipéridin-1-yl)pyrimidine-4-carboxamide, 2,0 g (0,00768 mole) de 3-(5-fluoro-2-méthoxyphénoxy)propylamine, 1,6 g (0,0115 mole) de carbonate de potassium et 30 ml de *N,N*-diméthylformamide et on porte le mélange réactionnel à une température de 70°C pendant 8 heures.
On le verse sur un mélange d'eau et de glace, on extrait à l'acétate d'éthyle, on lave la phase organique à l'eau, on la sèche et on la concentre sous pression réduite. On obtient une huile jaune et on prépare le chlorhydrate par réaction de 1,7 g (0,0042 mole) de base et de 42 ml d'acide chlorhydrique 0,1N dans du propan-2-ol. On évapore sous pression réduite et on fait recristalliser dans de l'éthanol.
On obtient 1,3 g de composé.
Point de fusion : 230-232,5°C.

Exemple 4 (Composé N°14)

2-[4-[[[2-[5-méthyl-2-(1-méthyléthyl)phénoxy]éthyl]amino]méthyl]pipéridin-1-yl]pyrimidine-4-carboxamide, chlorhydrate.

4.1 [[1-[4-(aminocarbonyl)pyrimidin-2-yl]pipéridin-4-yl]méthyl]carbamate de 1,1-diméthyléthyle.

Dans un ballon tricol de 1 litre on introduit 14 g (0,0653 mole) de [(pipéridin-4-yl)méthyl]carbamate de 1,1-diméthyle, 10,45 g (0,0663 mole) de 2-chloropyrimidine-4-carboxamide, 13,55 g (0,098 mole) de carbonate de potassium, 0,3 g d'iodure de sodium et 330 ml de *N,N*-diméthylformamide. On agite le mélange pendant 24 heures à température ambiante et sous atmosphère d'argon, puis on le verse dans 500 ml d'eau. On extrait au dichlorométhane, on lave la phase organique à l'eau, on la sèche sur sulfate de magnésium, on la filtre et on évapore le solvant sous pression réduite. On reprend le produit brut cristallisé avec de l'éther éthylique, et on obtient 20,4 g de solide blanc.
Point de fusion : 172-174,5°C.

4.2 2-[4-(aminométhyl)pipéridin-1-yl]pyrimidine-4-carboxamide.

Dans un ballon de 1 litre, on introduit 20,3 g (0,0605 mole) de [[1-[4-(aminocarbonyl)pyrimidin-2-yl]pipéridin-4-yl]méthyl]carbamate de 1,1-diméthyléthyle, 200 ml de dichlorométhane et 200 ml d'acide trifluoroacétique, et on chauffe le mélange à 40°C pendant 4,5 heures.

On dilue le mélange réactionnel avec 300 ml de dichlorométhane, on le refroidit à 0°C et on y fait passer un courant d'ammoniac gazeux. On élimine l'insoluble par filtration, on évapore le solvant sous pression réduite, on reprend le résidu avec du dichlorométhane, on sèche la solution sur sulfate de magnésium, on la filtre et on l'évapore sous pression réduite. On obtient 13,8 g d'amine déprotégée cristallisée, que l'on utilise telle quelle dans l'étape suivante.
Point de fusion : 144-148°C.

4.3. 2-[4-[[[2-[5-méthyl-2-(1-méthyléthyl)phénoxy]éthyl]amino]méthyl]pipéridin-1-yl]pyrimidine-4-carboxamide, chlorhydrate.

Sous argon, on met en suspension 3 g (0,0128 mole) de 2-[4-(aminométhyl)pipéridin-1-yl]pyrimidine-4-carboxamide, 3,3 g (0,0128 mole) de bromure de 2-[5-méthyl-2-(1-méthyléthyl)phénoxy]éthyle et 2,65 g (0,0192 mole) de carbonate de potassium dans 50 ml de *N*,*N*-diméthylformamide et on chauffe le mélange à 70°C pendant 9 heures.
On le refroidit à la température ambiante, on le verse sur 250 ml d'eau et on extrait avec de l'acétate d'éthyle. On lave la phase organique à l'eau, la sèche sur sulfate de magnésium, on la filtre et on évapore le solvant sous pression réduite.
Après purification par chromatographie sur silice (éluant : dichlorométhane/méthanol 96/4 à 90/10), on isole 1,85 g de base.
Pour préparer le chlorhydrate on dissout la base dans 50 ml de méthanol, on ajoute 45 ml d'acide chlorhydrique 0,1N dans le propan-2-ol et on évapore sous pression réduite. On recristallise le résidu d'évaporation dans l'éthanol pour obtenir 1,4 g de composé.
Point de fusion : 197-199,5°C.

Exemple 5 (Composé N°13)

2-[3-[[[2-(2-méthoxyphénoxy)éthyl]amino]méthyl]pipéridin1-yl]pyrimidine-4-carboxamide, chlorhydrate.

5.1. [[1-[4-(aminocarbonyl)pyrimidin-2-yl]pipéridin-3-yl]méthyl]carbamate de 1,1-diméthyléthyle.

Dans un ballon tricol de 1 litre on introduit 10 g (0,0467 mole) de [(pipéridin-3-yl)méthyl]carbamate de 1,1-diméthyléthyle, 7,5 g (0,0476 mole) de 2-chloropyrimidine-4-carboxamide, 9,7 g (0,07 mole) de carbonate de potassium, 0,3 g d'iodure de sodium et 230 ml de *N*,*N*-diméthylformamide. On agite le mélange pendant 24 heures à température ambiante, sous atmosphère d'argon, puis on le verse sur 500 ml d'eau. On extrait au dichlorométhane, on lave la phase organique à l'eau, on la sèche sur sulfate de magnésium, on la filtre et on évapore le solvant sous pression réduite. On reprend le produit brut cristallisé avec de l'éther de pétrole et on obtient 15 g de solide blanc.
Point de fusion : 131-135°C.

5.2. 2-[3-(aminométhyl)pipéridin-1-yl]pyrimidine-4-carboxamide.

Dans un ballon de 1 litre, on introduit 15 g (0,0447 mole) de [[1-[4-(aminocarbonyl)pyrimidin-2-yl]pipéridin-3-yl]méthyl]-carbamate de 1,1-diméthyléthyle, 150 ml de dichlorométhane et 150 ml d'acide trifluoroacétique, et on chauffe le mélange à 40°C pendant 5 heures.
On le dilue avec 230 ml de dichlorométhane, on le refroidit à 0°C et on y fait passer un courant d'ammoniac gazeux. On élimine l'insoluble par filtration, on évapore le solvant sous pression réduite, on reprend au dichlorométhane, on sèche la solution sur sulfate de magnésium, on la filtre et on évapore sous pression réduite. On obtient 10,2 g d'amine sous forme d'huile jaunâtre qu'on utilise telle quelle dans l'étape suivante.

5.3. 2-[3-[[[2-(2-méthoxyphénoxy)éthyl]amino]méthyl]pipéridin-1-yl]pyrimidine-4-carboxamide, chlorhydrate.

Sous argon, on met en suspension 3,5 g (0,0149 mole) de 2-[3-(aminométhyl)pipéridin-1-yl]pyrimidine-4-carboxamide, 3,5 g (0,0151 mole) de bromure de 2-(2-méthoxyphénoxy)éthyle et 3,1 g (0,0224 mole) de carbonate de potassium dans 60 ml de *N*,*N*-diméthylformamide, et on chauffe le mélange à 70°C pendant 12,5 heures.
On le refroidit à la température ambiante, on le verse sur 250 ml d'eau et on extrait avec de l'acétate d'éthyle. On lave la phase organique à l'eau, on la sèche sur sulfate de magnésium, on la filtre et on évapore le solvant sous pression réduite.

Après purification par chromatographie sur silice (éluant : dichlorométhane/méthanol 96/4 à 90/10), on isole 1,7 g de base.
Pour préparer le chlorhydrate on dissout la base dans 30 ml d'éthanol, on ajoute 44 ml d'acide chlorhydrique 0,1N dans le propan-2-ol, on évapore les solvants sous pression réduite et on recristallise le résidu dans le propan-2-ol.
On obtient finalement 1,3 g de composé.
Point de fusion : 199-201°C.

Exemple 6 (Composé N°1)

2-(4-aminopipéridin-1-yl)pyrimidine-4-carboxamide, chlorhydrate.

6.1. [1-[4-(aminocarbonyl)pyrimidin-2-yl]pipéridin-4-yl]carbamate de 1,1-diméthyléthyle.

Dans un ballon tricol de 500 ml on introduit 7,7 g (0,0385 mole) de (pipéridin-4-yl)carbamate de 1,1-diméthyléthyle, 6,15 g (0,039 mole) de 2-chloropyrimidine-4-carboxamide, 8 g (0,0578 mole) de carbonate de potassium, 0,3 g d'iodure de sodium et 200 ml de *N,N*-diméthylformamide, et on agite le mélange pendant 24 heures à température ambiante et sous atmosphère d'argon.
On le verse dans 500 ml d'eau, on l'extrait au dichlorométhane, on lave la phase organique à l'eau, on la sèche sur sulfate de magnésium, on la filtre et on évapore le solvant sous pression réduite. On reprend le produit brut cristallisé avec de l'éther diéthylique, et on obtient 11,8 g de solide blanc.
Point de fusion : 216,5-218°C.

6.2. 2-(4-aminopipéridin-1-yl)pyrimidine-4-carboxamide, chlorhydrate.

Dans un ballon tricol de 1 litre on introduit 13,9 g (0,0433 mole) de [1-[4-(aminocarbonyl)pyrimidin-2-yl]pipéridin-4-yl]-carbamate de 1,1-diméthyléthyle, 140 ml de dichlorométhane et 140 ml d'acide trifluoroacétique, et on chauffe le mélange pendant 4 heures à 40°C.
On dilue le mélange avec 300 ml de dichlorométhane, on le refroidit à 0°C, et on y fait passer un courant d'ammoniac gazeux. On élimine l'insoluble par filtration, on évapore le solvant sous pression réduite, on reprend le résidu avec du dichlorométhane, on sèche la solution sur sulfate de magnésium, on la filtre et on évapore le solvant sous pression réduite. On obtient 8,3 g d'amine déprotégée cristallisée.
Point de fusion : 105-107,5°C.
On en prépare le chlorhydrate au moyen d'acide chlorhydrique 0,1N dans le propan-2-ol.
Point de fusion : 304-307°C.

Exemple 7 (Composé N°5)

2-[4-[[2-(5-fluoro-5-méthoxyphénoxy)éthyl]amino]pipéridin-4-yl]pyrimidine-4-carboxamide, chlorhydrate.

Sous atmosphère d'argon on prépare une suspension de 0,6 g (0,00271 mole) de 2-(4-aminopipéridin-1-yl)pyrimidine-4-carboxamide, 0,68 g (0,00271 mole) de bromure de 2-(5-fluoro-2-méthoxyphénoxy)éthyle et 0,56 g (0,00407)mole) de carbonate de potassium dans 10 ml de *N,N*-diméthylformamide, et on l'agite à température ambiante pendant 24 heures, puis à 90°C pendant 3,5 heures.
On refroidit le mélange à température ambiante, on le verse sur 100 ml d'eau et on l'extrait avec de l'acétate d'éthyle. On lave la phase organique à l'eau, on la sèche sur sulfate de magnésium, on la filtre et on évapore le solvant sous pression réduite. On purifie le résidu d'évaporation par chromatographie sur colonne de gel de silice en éluant avec un mélange de dichlorométhane/méthanol 95/5 à 92/8, et on obtient 0,6 g de base.
Pour préparer le chlorhydrate, on dissout la base dans 10 ml de méthanol, on ajoute 15,5 ml d'acide chlorhydrique 0,1N dans le propan-2-ol, et on évapore les solvants sous pression réduite. On recristallise le résidu dans un mélange d'éthanol et de propan-2-ol, et on obtient finalement 0,4 g de chlorhydrate.
Point de fusion : 236-237,5°C.

Exemple 8 (Composé N°8)

2-[4-[[3-[4-fluorophénoxy)propyl]amino]pipéridin-1-yl]pyrimidine-4-carboxamide, chlorhydrate.

Sous atmosphère d'argon on prépare une suspension de 1,5 g (0,00678 mole) de 2-(4-aminopipéridin-1-yl)pyrimidine-4-carboxamide, 1,6 g (0,00678 mole) de bromure de 3-(4-fluorophénoxy) propyle et 1,4 g (0,0102 mole) de carbonate de potassium dans 25 ml de *N,N*-diméthylformamide, et on chauffe le mélange à 70°C pendant 7,5 heures.
On le refroidit à température ambiante, on le verse sur 250 ml d'eau et on l'extrait avec de l'acétate d'éthyle. On lave la phase organique à l'eau, on la sèche sur sulfate de magnésium, on la filtre et on évapore le solvant sous pression réduite.
On purifie le résidu par chromatographie sur colonne de gel de silice en éluant avec un mélange dichlorométhane/méthanol 95/5 à 90/10, et on isole 1,05 g de base.
Pour préparer le chlorhydrate on la dissout dans 20 ml de méthanol, on ajoute 28 ml d'acide chlorhydrique 0,1N dans le propan-2-ol, on évapore les solvants sous pression réduite, et on recristallise le résidu dans un mélange de méthanol et d'éthanol.
On isole finalement 0,6 g de chlorhydrate.
Point de fusion : 243-246°C.

Le tableau suivant illustre les structures chimiques et les propriétés physiques de quelques composés selon l'invention.

Tableau

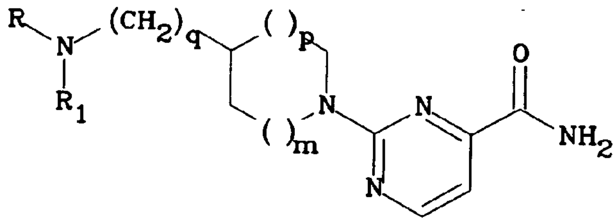

(I)

| N° | R | X | n | m,p | q | $R_1$ | Sel | F (°C) |
|---|---|---|---|---|---|---|---|---|
| 1 | H | - | - | 1,1 | 0 | H | HCl | 304-307 |
| 2 | $CH_3$ | - | - | 1,1 | 0 | $CH_3$ | HCl | 270-272 |
| 3 | 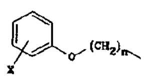 | 2-$OCH_3$ | 2 | 1,1 | 0 | H | HCl | 231-233 |
| 4 | 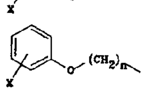 | 2-$OCH_3$, 5-Cl | 2 | 1,1 | 0 | H | HCl | 226-229 |
| 5 | 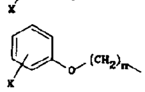 | 2-$OCH_3$, 5-F | 2 | 1,1 | 0 | H | HCl | 236-237,5 |
| 6 | 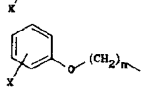 | 4-F | 2 | 1,1 | 0 | H | HCl | 229-231 |
| 7 | 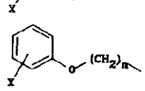 | 2-$OCH_3$, 5-F | 3 | 1,1 | 0 | H | HCl | 230-232,5 |
| 8 | 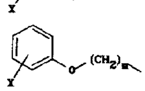 | 4-F | 3 | 1,1 | 0 | H | HCl | 243-246 |
| 9 | 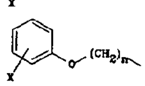 | 2-$iC_3H_7$, 5-$CH_3$ | 2 | 1,1 | 0 | H | HCl | 254-257 |
| 10 | 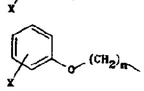 | 4-F | 2 | 1,1 | 1 | H | HCl | 237-239 |
| 11 | 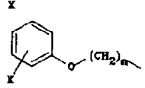 | 4-F | 2 | 0,2 | 1 | H | HCl | 184-187 |
| 12 | 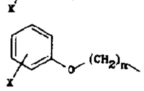 | 2-$OCH_3$ | 2 | 1,1 | 1 | H | HCl | 183-185 |
| 13 | 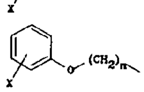 | 2-$OCH_3$ | 2 | 0,2 | 1 | H | HCl | 199-201 |
| 14 | 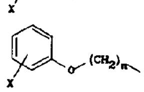 | 2-$iC_3H_7$, 5-$CH_3$ | 2 | 1,1 | 1 | H | HCl | 197-199,5 |

Note : dans la colonne "X", "$iC_3H_7$" désigne un groupe 1-méthyléthyle ; dans la colonne "Sel", "HCl" désigne un chlorhydrate.

Les composés de l'invention ont fait l'objet d'études quant à leur activité antagoniste des récepteurs $\alpha_1$-

adrénergiques au niveau du bas appareil urinaire.

Leur activité *in vitro* a été étudiée sur l'urètre isolé de lapin.

On prépare des anneaux d'urètre de lapin adulte selon la méthode de Ueda et al., *Eur. J. Pharmacol.*, (1984), **103**, 249-254, puis, après sensibilisation à la noradrénaline, on détermine la courbe concentration-réponse à la phényléphrine, en absence et en présence de composé à étudier.

On évalue la puissance de l'antagonisme $\alpha_1$-adrénergique de chaque composé par calcul du $pA_2$, antilogarithme de la concentration molaire d'antagoniste en présence de laquelle la concentration d'agoniste doit être doublée pour engendrer le même effet qu'en son absence.

Les $pA_2$ des composés sont de l'ordre de 5,5 à 9.

L'activité *in vivo* des composés de l'invention a été étudiée quant à leur effet sur l'hypertonie urétrale engendrée par la stimulation des fibres sympathiques du nerf hypogastrique chez le chat anesthésié.

On anesthésie des chats mâles adultes au pentobarbital sodique, et on les prépare selon la méthode de *Theobald, J. Auton. Pharmac.*, (1983), **3**, 235-239, afin d'obtenir une hypertonie urétrale par stimulation des fibres sympathiques du nerf hypogastrique. On note les réponses contractiles de l'urètre à la stimulation électrique du nerf hypogastrique avant et après administration intraveineuse des composés à étudier, à doses cumulatives de 1 à 1000 µg/kg.

On évalue la puissance de l'antagonisme $\alpha_1$-adrénergique de chaque composé par calcul de la $DI_{50}$, dose qui inhibe de 50% l'hypertonie urétrale.

Les $DI_{50}$ des composés de l'invention sont de l'ordre de 0,01 à 1 mg/kg.

Les résultats des essais montrent que les composés de l'invention montrent, *in vitro*, une activité antagoniste des récepteurs $\alpha_1$-adrénergiques des muscles lisses du bas appareil urinaire (urètre) stimulés par un agoniste $\alpha_1$-adrénergique (phényléphrine). *In vivo*, ils inhibent l'hypertonie urétrale engendrée par la stimulation nerveuse sympathique.

Les composés de l'invention peuvent donc être utilisés pour le traitement symptomatique des maladies et affections impliquant une hyperactivité du système $\alpha$-adrénergique au niveau du bas appareil urinaire, et notamment pour le traitement de l'hypertrophie bénigne de la prostate, de la dysurie, de la pollakiurie.

A cet effet ils peuvent être présentés sous toutes formes appropriées à l'administration entérale ou parentérale, associés à des excipients pharmaceutiques, par exemple sous forme de comprimés, dragées, gélules, capsules, solutions ou suspensions buvables ou injectables, suppositoires, étant dosés pour permettre une dose journalière de 0,5 à 500 mg de substance active.

## Revendications

1. Composé, sous forme d'énantiomère pur ou de mélange d'énantiomères, répondant à la formule générale (I)

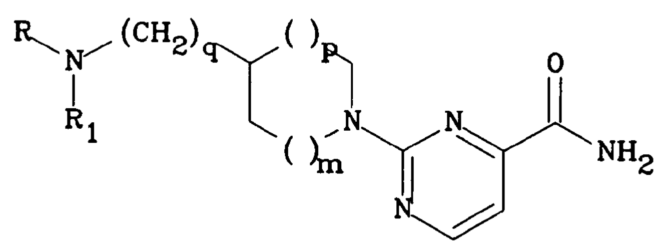

(I)

dans laquelle

R représente un atome d'hydrogène ou un groupe méthyle ou un groupe phénoxyalkyle de formule générale

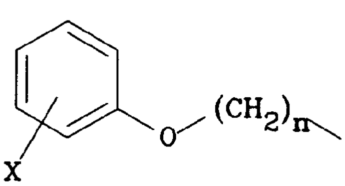

dans laquelle

X représente un ou plusieurs substituants choisis parmi les atomes d'hydrogène, de fluor et de chlore et les groupes méthyle, 1-méthyléthyle et méthoxy, et

n représente le nombre 2 ou 3,

m représente le nombre 1, auquel cas p représente le nombre 1, ou bien

m représente le nombre 0, auquel cas p représente le nombre 2,

q représente le nombre 0 ou 1, et

$R_1$ représente un atome d'hydrogène ou un groupe méthyle, à l'état de base ou de sel d'addition à un acide.

2. Composé selon la revendication 1, caractérisé en ce que R représente un groupe de formule générale

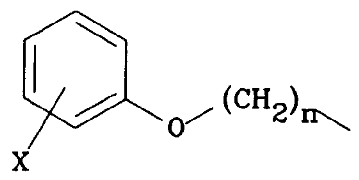

n représente le nombre 2, m représente le nombre 1, p représente le nombre 1, et q représente le nombre 0.

3. Procédé de préparation d'un composé selon la revendication 1, caractérisé en ce qu'on fait d'abord réagir une amine de formule générale (II)

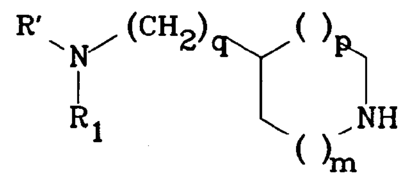

(II)

dans laquelle $R_1$, m, p et q sont tels que définis ci-dessus et R' représente un groupement protecteur de l'amine, avec le 2-chloropyrimidine-4-carboxamide, dans un solvant aprotique, en présence d'une base, pour obtenir une aminopyrimidine de formule générale (IV)

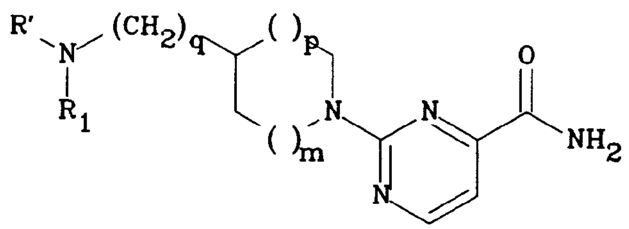

(IV)

que l'on déprotège ensuite, pour obtenir une aminopyrimidine de formule générale (V)

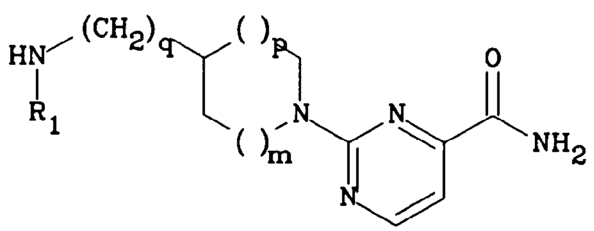

(V)

et, si on le désire, on fait finalement réagir cette dernière avec un halogénure de phénoxyalkyle de formule générale (VI)

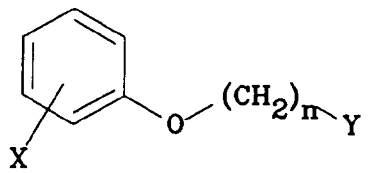

(VI)

dans laquelle X et n sont tels que définis ci-dessus et Y représente un atome de chlore ou de brome, dans un solvant aprotique et en présence d'une base.

4. Médicament caractérisé en ce qu'il consiste en un composé selon la revendication 1.

5. Composition pharmaceutique caractérisée en ce qu'elle contient un composé selon la revendication 1, en association avec tout excipient approprié.

**Revendications pour les Etats contractants suivants: ES, GR**

1. Procédé de préparation d'un composé répondant à la formule générale (I)

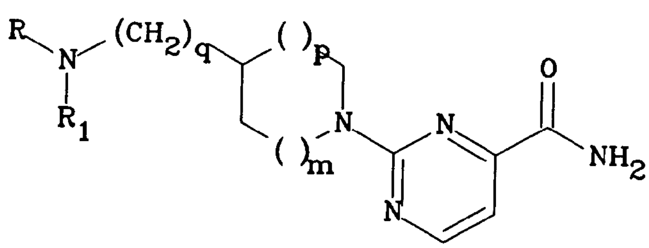

(I)

dans laquelle

R représente un atome d'hydrogène ou un groupe méthyle ou un groupe phénoxyalkyle de formule générale

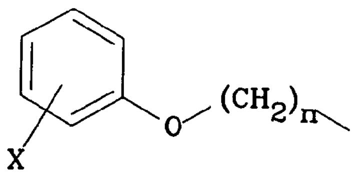

dans laquelle

X représente un ou plusieurs substituants choisis parmi les atomes d'hydrogène, de fluor et de chlore et les groupes méthyle, 1-méthyléthyle et méthoxy, et

n représente le nombre 2 ou 3,

m représente le nombre 1, auquel cas p représente le nombre 1, ou bien

m représente le nombre 0, auquel cas p représente le nombre 2,

q représente le nombre 0 ou 1, et

$R_1$ représente un atome d'hydrogène ou un groupe méthyle, procédé caractérisé en ce qu'on fait d'abord réagir une amine de formule générale (II)

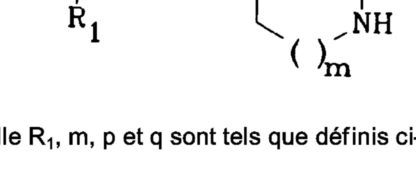

(II)

dans laquelle $R_1$, m, p et q sont tels que définis ci-dessus et R′ représente un groupement protecteur de

l'amine, avec le 2-chloropyrimidine-4-carboxamide, dans un solvant aprotique, en présence d'une base, pour obtenir une aminopyrimidine de formule générale (IV)

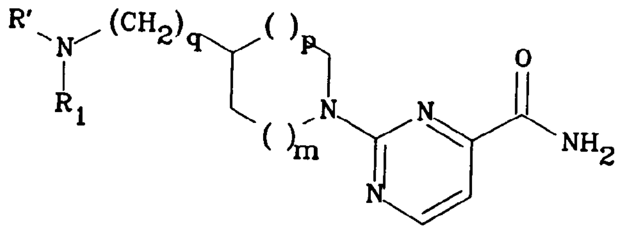

(IV)

que l'on déprotège ensuite, pour obtenir une aminopyrimidine de formule générale (V)

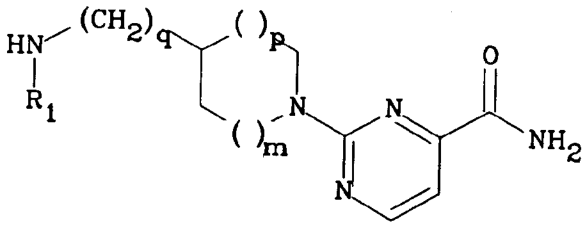

(V)

et, si on le désire, on fait finalement réagir cette dernière avec un halogénure de phénoxyalkyle de formule générale (VI)

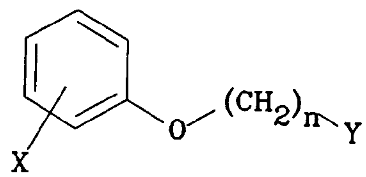

(VI)

dans laquelle X et n sont tels que définis ci-dessus et Y représente un atome de chlore ou de brome, dans un solvant aprotique et en présence d'une base.

Office européen des brevets

# RAPPORT DE RECHERCHE EUROPEENNE

Numero de la demande

EP 92 40 1770

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 301 936 (SYNTHELABO)<br>* revendications *<br>----- | 1,4,5 | C07D239/42<br>C07D401/04<br>C07D401/12<br>A61K31/505 |
| | | | **DOMAINES TECHNIQUES RECHERCHES (Int. Cl.5 )** |
| | | | C07D |

Le présent rapport a été établi pour toutes les revendications

| Lieu de la recherche | Date d'achèvement de la recherche | Examinateur |
|---|---|---|
| LA HAYE | 21 SEPTEMBRE 1992 | FRANCOIS J.C. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)